# FASCICULE DE BREVET EUROPEEN

(11) **EP 0 826 657 B1**
(45) Date de publication et mention de la délivrance du brevet: **01.08.2001**
(21) Numéro de dépôt: 97401999.4
(22) Date de dépôt: 27.08.1997
(51) Int. Cl.: C07C 47/277, C07C 59/64, C07C 69/734, C07C 43/23, C07C 235/34, C07D 295/18, C07D 311/58, C07D 335/06, A61K 31/085, A61K 31/335, A61K 31/38, A61K 7/00

(54) **Nouveaux composés modulateurs des récepteurs hormonaux, les compositions les comprenant et leur utilisation en thérapie**
Verbindungen zur Modulierung von Hormonrezeptoren, diese enthaltende Zusammensetzungen und ihre Verwendung in Therapie
Compounds for modulation of hormone receptors, compositions containing them and their use in therapy

(30) Priorité: 02.09.1996 FR 9610686
(43) Date de publication de la demande: 04.03.1998
(73) Titulaire: CENTRE INTERNATIONAL DE RECHERCHES DERMATOLOGIQUES GALDERMA, ( CIRD GALDERMA), 06560 Valbonne (FR)
(72) Inventeur: Thoreau, Etienne, 06460 San Vallier de They (FR); Shroot, Braham, 06600 Antibes (FR)
(74) Mandataire: Tezier Herman, Béatrice

(56) Documents cités:
- EP-A- 0 568 898
- WO-A-93/21146
- WO-A-95/04036
- FR-A- 2 390 428
- KOCH S S C ET AL: "Identification of the First Retinoid X Receptor Homodimer Antagonist" J. MED. CHEM. (JMCMAR,00222623);96; VOL.39 (17); PP.3229-3234, LIGAND PHARMACEUTICALS INC.;DEPARTMENT OF RETINOID CHEMISTRY RESEARCH; SAN DIEGO; 92121; CA; USA (US), XP002030143
- THACHER, SM: "Therapeutic applications for ligands of retinoid receptors", CURR. PHARM. DES., , 2000, Vol. 6, no. 1, pages 25 à 58
- ZHOU MD: "Retinoid-dependent pathways suppress myocardial cell hypertrophy", PROC NATL ACAD SCI, , 1995, Vol. 92, no. 16, pages 7391 à 7395
- GOTTARDIS MM: "Chemoprevention of mammary carcinoma by LGD1069 (Targretin): an RXR selective", CANCER RES, , 1996, Vol. 56, no. 24, pages 5566 à 5570
- BILLONI N: "Expression of retinoid nuclear receptor superfamily members in human hair follicles and its implication in hair growth", ACTA DERM VENEREOL, , 1997, Vol. 77, no. 5, pages 350 à 355

## Description

La présente invention concerne de nouveaux composés modulateurs des familles de récepteurs cellulaires hormonaux tels que ceux décrits par Guiguère ou Evans (Guiguère V., Endocrine Reviews, 15,1, 1994,61; Mangelsdorf D. & Evans R., Cell, 83, 1995, 841).

Compte tenu de cette interaction, ces nouveaux composés exercent une activité biologique sur les cellules et tissus animaux au regard de leur différentiation, de leur prolifération ou leur implication dans la mort cellulaire (apoptose). Ces substances agissent en combinaison avec des ligands connus de la super famille, par exemple l'acide rétinoïque et les rétinoïdes, la vitamine D3, l'hormone thyroïdienne, les oestrogènes, etc., de manière à améliorer leur rapport risque / bénéfice, tant en réduisant leur toxicité qu'en améliorant leur efficacité pharmacologique.

Les composés selon l'invention permettent la prévention et/ou le traitement de désordres et/ou d'affections liés à une surrégulation des récepteurs liés à ces hormones ou vitamines. Ils peuvent également être impliqués dans le contrôle des différentes étapes du métabolisme ou du catabolisme de ces hormones ou vitamines.

Le document EP-A-0 568 898 décrit des composés présentant une activité antagoniste des récepteurs RARα. Ces composés peuvent présenter une structure de formule (I) dans laquelle X représente -S-, mais qui diffèrent des composés de l'invention par le radical correspondant au radical R1 qui n'est pas substitué.

La présente invention concerne donc de nouveaux composés de formule générale (I) 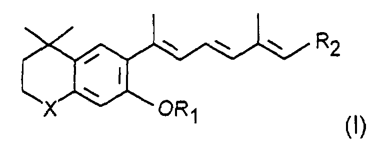 dans laquelle
X représente un radical divalent >C(CH₃)₂, un atome d'oxygène (-O-) ou un atome de soufre (-S-),
R₁ représente un radical de formule -(CH₂)ₙ-Z-(CH₂)ₒ-Y, Z, Y, n et o étant définis ci-dessous,
Y représente OH, un radical thiol (-SH), un radical triméthylammonium (-N⁺(CH₃)₃), un radical cyano (-CN), un radical -COR₃, ou un radical -NH2, R₃ étant défini ci-dessous,
Z représente un radical méthylène (-CH₂-) ou un atome d'oxygène (-O-),
R₂ représente un radical -COR₃ ou -CH₂-OH,
R₃ représente un atome d'hydrogène, un radical hydroxyle, un radical alkoxyle ayant de 1 à 6 atomes de carbone ou cycloalkoxyle, ou encore un radical de formule -NR₄R₅,
R₄ et R₅, identiques ou différents, représentent indépendamment un atome d'hydrogène ou un radical alkyle ayant de 1 à 6 atomes as carbone, ou R₄ et R₅ forment ensemble un hétérocycle avec l'atome d'azote auquel ils sont rattachés,
n et o, identiques ou différents, représentent indépendamment un entier compris entre 1 et 5,
leurs racémiques ou leurs isomères optiques ou géométriques purs ou leurs mélanges en toutes proportions, et leurs sels pharmaceutiquement acceptables.

Lorsque les composés selon l'invention se présentent sous forme de sels, il s'agit de préférence de sels d'un métal alcalin ou alcalino-terreux ou encore de zinc ou d'une amine organique.

Selon la présente invention, on entend par radical alkyle inférieur un radical ayant de 1 à 6 atomes de carbone, linéaire ou ramifié, de préférence les radicaux méthyle, éthyle, isopropyle, butyle, tertiobutyle et hexyle. Cette définition s'applique également aux restes alkyles des radicaux alkoxyles.

Par cycloalkoxyle on entend de préférence selon l'invention les radicaux dont le reste cycloalkyle est constitué par un groupe mono ou polycyclique, comprenant 3 à 12 atomes de carbone, éventuellement substitués par un ou plusieurs radicaux alkyle inférieurs.

Par hétérocycle, on entend de préférence un radical pipéridino, morpholino, pyrrolidino ou pipérazino, éventuellement substitué en position 4 par un radical alkyle inférieur.

D'une manière préférentielle, lorsque Z représente un atome d'oxygène (-O-), alors Y représente un radical cyano (-CN) ou un radical -COR₃, R₃ étant défini ci-dessus, n représente 2, 3 ou 4 et o représente 1, 2 ou 3.

Selon un autre mode préférentiel de réalisation de l'invention, lorsque Z représente un radical méthylène (-CH₂-), alors Y représente un radical -thiol (-SH), triméthylammonium (-N⁺(CH₃)₃) ou cyano (-CN).

Ces composés de formule (I) peuvent être notamment obtenus en faisant réagir un composé hydroxylé de formule (II) 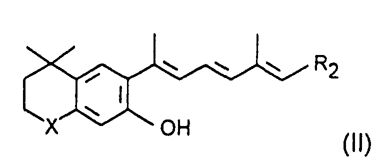 dans laquelle X et R₂ sont définis ci-dessus, avec un composé de formule (III)

X-(CH₂)ₙ-Z-(CH₂)ₒ-Y (III)

dans laquelle Z, Y, n et o sont définis ci-dessus, et X représente un groupe partant, comme un halogène, en particulier un brome ou l'iode.

Le composé hydroxylé de formule (II) est préparé à partir du dérivé alkoxylé correspondant décrit dans la demande de brevet DE 28 19 213, notamment le dérivé méthoxylé décrit en exemple 9. L'hydrolyse de la fonction éther est réalisée selon les méthodes usuelles de la technique, avec protection et déprotection appropriée des fonctions sensibles aux conditions d'hydrolyse.

En particulier, lorsque R₂ représente le radical -COOH, les composés sont préparés en protégeant R₂ par un groupe protecteur de type alkyle, allylique, benzylique ou tert-butylique.

Le passage à la forme libre peut être effectué:
- dans le cas d'un groupe protecteur alkyle, au moyen de soude ou d'hydroxyde de lithium dans un solvant alcoolique tel le méthanol ou dans le THF.
- dans le cas d'un groupe protecteur allylique, au moyen d'un catalyseur tel certains complexes de métaux de transition en présence d'une amine secondaire telle la morpholine.
- dans le cas d'un groupement protecteur benzylique, par débenzylation en présence d'hydrogène au moyen d'un catalyseur tel que le palladium sur charbon.
- dans le cas d'un groupement protecteur de type tert-butylique au moyen d'iodure de triméthylsilane.

D'une manière avantageuse, on transforme le phénol de formule générale (Il) en un composé de formule (I) avec le radical de formule (III) en présence de carbonate de potassium (K2CO3) dans le méthyl éther cétone (MEK) ou en présence d'hydrure de sodium dans un solvant tel que le diméthylformamide (DMF).

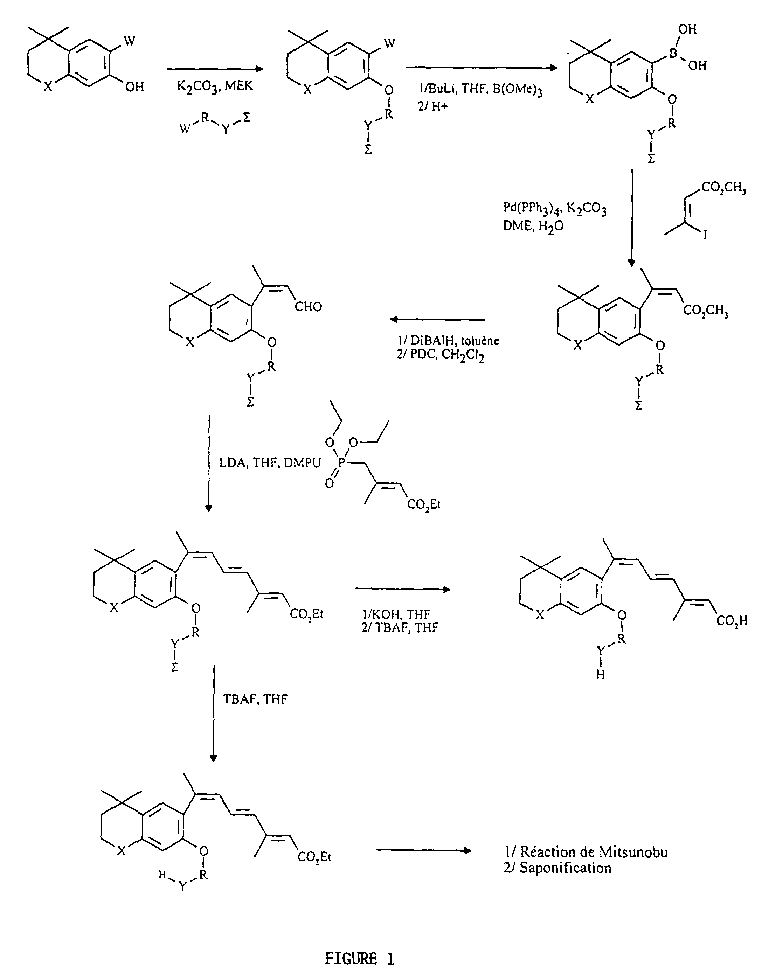

Ces composés peuvent être également obtenus selon le schéma réactionnel donné dans la figure 1. Dans cette figure : W représente un atome d'halogène tel que le brome ou l'iode, Σ représente un groupe protecteur, tel que le terbutyldiméthylsilane (TBDMSi), R le radical -(CH2)n-Z-(CH2)o- et Y est tel que dans la demande. Les réactions 1/ de Mitsunobu et 2/ de saponification sont éventuellement réalisées si Y représente un atome d'oxygène et on désire le transformer en atome de soufre ou d'azote. Les exemples ci-dessous illustrent ce schéma réactionnel.

La présente invention concerne également l'utilisation des composés selon l'invention à titre de médicament et les compositions cosmétiques ou pharmaceutiques comprenant au moins un composé de formule générale (I) tel que défini ci-dessus et au moins un véhicule cosmétiquement ou pharmaceutiquement acceptable.

Les compositions cosmétique ou pharmaceutique contenant au moins un composé de formule (I) sont en particulier destinées au traitement de désordres ou d'affections liés à une surrégulation des facteurs de transcription dénommés les récepteurs rétinoïdes qui comprennent les Récepteurs Rétinoïques X (RXRs), y compris les sous-types α, β, γ, ou les gènes comprenant les éléments de réponse RXRs.

Par surrégulation du récepteur RXRs, on entend selon l'invention une surexpression du récepteur RXRs, et/ou une suractivité biologique du récepteur RXRs.

La suractivité biologique du récepteur RXRs peut être due à une modification chimique du récepteur RXRs, mais elle peut être due également à un facteur autre que le récepteur lui-même. Ainsi, la suractivité biologique peut être due à la surexpression d'un gène endogène ou à l'expression d'un gène exogène comprenant l'élément de réponse RARE (retinoic acid response element) sur lequel est venu se fixer un hétérodimère comprenant le récepteur RAR, ce dernier portant un ligand agoniste. A titre d'exemple de surexpression d'un gène endogène comprenant l'élément de réponse RARE, on peut citer le gène de la CRABP II (cellular retinoic acid binding protein Il) dont la surexpression a été démontrée dans le psoriasis ("Overexpression of CRABP Il and down-regulation of CRABP I in psoriatic skin", G. Siegenthaler et al., Dermatology 1992; 185:251-256). A titre d'exemple d'expression d'un gène exogène comprenant l'élément de réponse RARE, on peut citer le génome HIV-1 (virus d'immunodéficience humain) (Proc. Natl. Acad. Sci. USA, Lee et al., Vol.91, pp. 2632-5636, June 1994) ou le génome du virus de l'hépatite B ("Retinoid X receptor RXR alpha binds to and trans-activates the hepatite B virus enhancer", B. Huan et al., Proc. Natl. Acad. Sci. USA, 1992, 89 (19), p 9059-63).

Ces désordres et/ou affections liés à une surrégulation des récepteurs RARs et/ou RXRs se traduisent le plus souvent par une composante inflammatoire, allergique et/ou immunologique. Ils sont plus particulièrement présents dans les pathologies ou les désordres suivants :
1) l'acné,
2) les affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique,
3) certaines affections inflammatoires ne présentant pas de trouble de la kératinisation, telles que l'arthrite,
4) les proliférations dermiques ou épidermiques malignes,
5) le vieillissement de la peau, qu'il soit photo-induit ou chronologique ou les pigmentations et les kératoses actiniques, ou toutes pathologies associées au vieillissement chronologique ou actinique,
6) les troubles de la fonction sébacée tels que l'hyperséborrhée de l'acné ou la séborrhée simple,
7) états cancéreux ou précancéreux,
8) affection d'origine virale au niveau cutané ou général (virus d'immunodéficience humain : HIV-1 ou virus de l'hépatite B),
9) l'alopécie,
10) affections du système cardio-vasculaire,

Dans le cadre de l'invention, les composés de formule (1) peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinone ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

Les compositions cosmétique ou pharmaceutique comprenant une quantité efficace d'au moins un composé de formule (I), l'un de ses analogues chiraux ou encore l'un de ses sels comprend un support cosmétiquement ou pharmaceutiquement acceptable et compatible avec le mode d'administration retenu.

La quantité efficace dépendant bien entendu du traitement désiré et de la nature du composé choisi est donc déterminée par l'homme du métier.

L'administration des composés selon l'invention peut être effectuée par voie entérale, parentérale, topique ou oculaire.

Par voie entérale, la composition, plus particulièrement la composition pharmaceutique, peut se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, la composition, plus particulièrement la composition pharmaceutique, peut se présenter sous forme de solutions ou de suspensions pour perfusion ou pour injection.

Les composés selon l'invention sont généralement administrés à une dose journalière d'environ 0,01 mg/kg à 100 mg/kg en poids corporel, et ceci à raison de 1 à 3 prises.

Par voie topique, la composition est plus particulièrement destinée au traitement de la peau et des muqueuses et peut alors se présenter sous forme d'onguents, de crèmes, de laits, de pommades, de poudres, de tampons imbibés, de solutions, de gels, de sprays, de lotions, de suspensions ou de shampooings. Il peut également se présenter sous forme de microsphères ou nanosphères ou vésicules lipidiques ou polymériques ou de patches polymériques et d'hydrogels permettant une libération contrôlée. Cette composition par voie topique peut par ailleurs se présenter soit sous forme anhydre, soit sous une forme aqueuse.

Par voie oculaire, ce sont principalement des collyres.

Cette composition à usage topique ou oculaire contient au moins un composé de formule (I) telle que définie ci-dessus, ou l'un de ses analogues chiraux ou encore l'un de ses sels, à une concentration de préférence comprise entre 0,001% et 5% en poids par rapport au poids total de la composition.

La composition selon l'invention peut en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3- méthyl 1,2,4-benzothiadiazine 1,1-dioxyde) et le Phénytoïn (5,5-diphényl-imidazolidine 2,4-dione); des agents anti-inflammatoires non stéroïdiens; des caroténoïdes et, notamment, le β-carotène; des agents anti-psoriatiques tels que l'anthraline et ses dérivés; et enfin les acides eicosa-5,8,11,14-tétraynoïque et eicosa-5,8,11-triynoïque, leurs esters et amides.

La composition peut également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

### EXEMPLE 1: Acide (E, E, Z)-7-[3-(5-Hydroxy-pentyloxy)-5,5,8,8-tétraméthyl-5,6,7,8-tétrahydro-naphthalène-2-yl]-3-méthyl-octa-2,4,6-triènoïque de formule

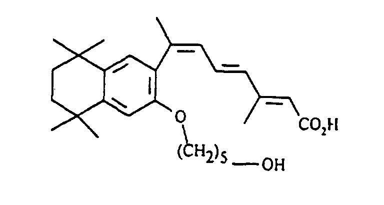

### Acétate de 5-(3-bromo-5,5,8,8-tétraméthyl-5,6,7,8-tétrahydro-naphthalène-2-yloxy)-pentyl.

Une solution de 3-bromo-5,5,8,8-tétraméthyl-5,6,7,8-tétrahydronaphtalène-2-ol (10g, 0,35 mol), de 5-bromopentylacétate (8,15 g), et de carbonate de potassium (33,6 g) dans la méthyléthylcétone (MEK) (200 ml), est chauffée à reflux pendant 2 heures. Le milieu réactionnel est traité par de l'eau et de l'acétate d'éthyle. Après décantation, la phase organique est lavée deux fois à l'eau, séchée sur sulfate de magnésium et concentrée à l'évaporateur rotatif sous vide à 40 °C. Le produit est purifié par chromatographie flash sur colonne de silice. Huile jaune. Rendement: 93%.

### [5-(3-Bromo-5,5,8,8-tétraméthyl-5,6,7,8-tétrahydro-naphthalène-2-yloxy)-pentyloxy)-tert-butyl-diméthyl-silane

L'acétate précédemment obtenu est saponifié puis le groupement hydroxyle résultant est protégé selon le mode opératoire suivant: du chlorure de tert-butyldiméthylsilyl (2,64 g) est additioné à un mélange de 5-(3-bromo-5,5,8,8-tétraméthyl-5,6,7,8-tétrahydro-naphthalène-2-yloxy)-pentan-1-ol (4,3g, 11,7 mmol) et d'hydrure de sodium à 80% (422 mg) dans le THF (20ml).
Le mélange est agité à température ambiante, 2 h. La solution est versée dans un mélange d'eau et d'acétate d'éthyle. La phase organique est lavée deux fois par de l'eau, séchée sur sulfate de magnésium, et concentrée à l'évaporateur rotatif sous vide à 40°C. Le produit est purifié par chromatographie flash sur colonne de silice.
Huile jaune. Rendement: 64%.

### Acide 3-[5-(tert-butyl-diméthyl-silanyloxy)-pentyloxy]-5,5,8,8-tétraméthyl-5,6,7,8-tétrahydro-naphthalène-2-boronique

Une solution de butyl lithium (BuLi) dans l'hexane 2,5 M (0,45 ml, 1,12 mmol) est additionnée goutte à goutte à une solution du produit précédemment obtenu (500 mg, 1,03 mmol), dans le THF (2 ml) à - 78°C. L'agitation est poursuivie 0,5h à la même température puis le triméthoxyborate (B(OMe)3) (0,35 ml) est additionné goutte à goutte à78°C. L'agitation est poursuivie 3h à -78°C puis le milieu réactionnel est hydrolysé à -40°C par une solution de NH₄Cl saturée. Le mélange est extrait à l'éther éthylique, lavé 2 fois à l'eau, séché et concentré à l'évaporateur rotatif sous vide. Huile incolore. Rendement: 75%.

### (Z)-3-{3-[5-(tert-butyl-diméthyl-silanyloxy)-pentyloxy]-5,5,8,8-tétraméthyl-5,6,7,8-tétrahydro-naphthalène-2-yl}-but-2-ènoate de méthyle.

Un mélange de tétrakis(triphénylphosphine)palladium (Pd(PPh3)4) (155 mg), d'acide 3-[5-(*tert*-butyl-diméthyl-silanyloxy)-pentyloxy]-5,5,8,8-tétraméthyl-5,6,7,8-tétrahydro-naphthalène-2-boronique (300mg, 0,67 mmol), d'une solution 2M de carbonate de potassium dans l'eau (0,67 ml) et de (Z)-3-lodo-2-butènoate de méthyl (Ma, S.; Lu, X.; J. Chem. Soc. Chem. Com. 1990, pp 1643-1644) (182 mg) dans le diméthoxyéthane (DME) (10 ml) est chauffé à reflux pendant 24h. La solution est versée dans un mélange d'eau et d'acétate d'éthyle. La phase organique est lavée deux fois par de l'eau, séchée sur sulfate de magnésium, et concentrée à l'évaporateur rotatif sous vide à 40°C. Le produit est purifié par chromatographie flash sur colonne de silice.
Huile incolore. Rendement: 80%.
RMN d ppm: 1H/CDCl₃: -0.012 (s, 6H); 0.92 (s, 9H); 1.18 à 1.25 (m,12H); 1.36 à 1.57 (m, 4H); 1.60 (s,3H); 1.65 à 1.74 (m, 2H); 2.12 (d, 3H); 3.45 (s, 3H); 3.57 (t, 2H); 3.87 (t, 3H); 5.85 (d, 1H); 6.69 (s, 1H); 6.88 (s, 1H).

### (Z)-3-(3-[5-(tert-butyl-diméthyl-silanyloxy)-pentyloxy]-5,5,8,8-tétraméthyl-5,6,7,8-tétrahydro-naphthalène-2-yl}-but-2-enal

Une solution 1M d'hydrure de diisobutylaluminium (DiBAIH) dans le toluène (3,8 ml) est additionnée goutte à goutte à 0°C à une solution de (Z)-3-{3-[5-(*tert*-butyl-diméthyl-silanyloxy)-pentyloxy]-5,5,8,8-tétraméthyl-5,6,7,8-tétrahydro-naphthalène-2-yl}-but-2-ènoate de méthyle.(1,9 g, 37,8 mol) dans le toluène (40 ml). Le mélange est agité 1h à température ambiante puis traité par une solution de tartrate double de sodium/potassium, filtré. Le filtrat est versé dans un mélange éther éthylique/eau. La phase organique est lavée à l'eau, séchée sur sulfate de magnésium anhydre, concentrée à l'évaporateur rotatif sous vide à 40°C. L'huile résultante est solubilisée dans 30 ml de dichlorométhane et 2g de pyridinium dichromate (PDC) sont additionnés. Le mélange est agité 4h à température ambiante, filtré sur silice. Le filtrat est concentré à l'évaporateur rotatif sous vide à 40°C et purifié par chromatographie flash sur colonne de silice.
Huile incolore. Rendement: 61%.
RMN d ppm: 1H/CDCl₃: 0.00 (s, 6H); 0.84 (s, 9H); 1.18 à 1.25 (m, 12H); 1.44 à 1.50 (m, 4H); 1.63 (s, 3H); 1.66 à 1.74 (m, 2H); 2.23 (d, 3H); 3.57 (t, 2H); 3.89 (t, 3H); 6.04 (d, 2H/J=6.96); 6.74 (s, 1H); 6.94 (s, 1H); 9.31(d, 1H).

### (E, E, Z)-7-{3-[5-(tert-butyl-diméthyl-silanyloxy)-pentyloxy]-5,5,8,8-tétraméthyl-5,6,7,8-tétrahydro-naphthalène-2-yl}-3-méthyl-octa-2,4,6-triènoate d'éthyle

Une solution de BuLi dans l'hexane 2,5 M (1,12) est additionnée goutte à goutte à une solution de lithium de diisopropylamine (LDA) (387 µl), dans un mélange de 1,3-diméthyl-3,4,5,6-tétrahydro-2(*1H*)-pyrimidinone (DMPU)/THF (10ml/10ml) à -20°C. Le mélange est agité 30 mn à -20°C, puis le triéthyl 3-méthyl-4-phosphonocrotonate (0,68 ml) est additionné. Le mélange est agité 1h à -20°C et est additionné sur une solution de (Z)-3-{3-[5-(*tert*-butyl-diméthyl-silanyloxy)-pentyloxy]-5,5,8,8-tétraméthyl-5,6,7,8-tétrahydro-naphthalène-2-yl}-but-2-ènal (1,1g, 2,3mmol) dans le THF (5ml). Le milieu réactionnel est laissé revenir à température ambiante puis traité à l'acétate d'éthyle/eau. Après décantation la phase organique est lavée deux fois à l'eau, séchée sur sulfate de magnésium anhydre et concentré à l'évaporateur rotatif sous vide à 40°C. Le produit est purifié par chromatographie flash sur colonne de silice.
Huile incolore. Rendement: 84%.
RMN d ppm: 1H/CDCl₃: 0.00 (s, 6H); 0.85 (s, 9H); 1.19 à 1.25 (m, 15H); 1.39 à 1.49 (m, 4H); 1.64 (s, 4H); 1.69 à 1.82 (m,2H); 2.09 (s, 3H); 2.14 (s, 3H); 3.57 (t, 2H); 3.90 (t, 2H); 4.12 (q, 2H); 5.68 (s,1H); 6.13 à 6.19 (m, 2H); 6.51 à 6.62 (m,1H) 6.75 (s, 1H); 6.92(s,1H)

### Acide (E, E, Z)-7-{3-[5-(tert-butyl-diméthyl-silanyloxy)-pentyloxy]-5,5,8,8-tétraméthyl-5,6,7,8-tètrahydro-naphthalen-2-yl}-3-méthyl-octa-2,4,6-triènoïque.

Une solution de (E, E, Z)-7-{3-[5-(*tert*-butyl-diméthyl-silanyloxy)-pentyloxy]-5,5,8,8-tétraméthyl-5,6,7,8-tétrahydro-naphthalen-2-yl}-3-méthyl-octa-2,4,6-triènoate d'éthyle (880 mg), d'hydroxyde de potassium (880mg) dans 22 ml d'un mélange THF, méthanol et eau (20, 1, 1) est chauffée à reflux pendant 4 h. Après concentration à l'évaporateur rotatif sous vide à 40°C, de l'acétate d'éthyle et de l'eau sont additionnés. Le mélange est acidifié jusqu'à pH 1 par une solution d'acide chlorhydrique concentré. Après décantation la phase organique est lavée deux fois à l'eau, séchée sur sulfate de magnésium anhydre et concentrée à l'évaporateur rotatif sous vide à 40°C.
Huile incolore. Rendement: 100%.
RMN d ppm: 1H/CDCl₃: 0.00 (s, 6H); 0.85 (s, 9H); 1.22 à 1.26 (m, 15H); 1.38 à 1.51(m,4H); 1.55 (s, 4H); 1.63 à 1.79 (m, 2H); 2.05 (s, 3H); 2.15 (s, 3H); 3.57 (t, 2H); 3.89 (t, 2H); 5.70(s, 1H); 6.16 à 6.22 (m, 2H); 6.56 à 6.66 (q, 1H); 6.74 (s, 1H); 6.91(s, 1H)

### Acide (E, E, Z)-7-[3-(5-Hydroxy-pentyloxy)-5,5,8,8-tétraméthyl-5,6,7,8-tétrahydro-naphthalène-2-yl]-3-méthyl-octa-2,4,6-triènoïque

Un mélange d'acide (E, E, Z)-7-{3-[5-(*tert*-butyl-diméthyl-silanyloxy)-pentyloxy]-5,5,8,8-tétraméthyl-5,6,7,8-tétrahydro-naphthalène-2-yl}-3-méthyl-octa-2,4,6-triènoïque (850mg, 1,5mmol), d'une solution 1M dans le THF de fluorure de tétrabutylammonium (TBAF) (3,1ml) dans le THF (10ml) est agité à température ambiante pendant 1 h puis traité à l'acétate d'éthyle et à l'eau. Après décantation la phase organique est lavée deux fois à l'eau, séchée sur sulfate de magnésium anhydre et concentrée à l'évaporateur rotatif sous vide à 40°C.
RMN d ppm: 1H/CDCl₃: 1.23 à 1.30 (m, 12H); 1.48 à 1.65 (m, 4H); 1.68 (s, 4H); 1.73 à 1.86 (m,2H); 2.14 (s, 3H); 2.18 (s, 3H); 3.65 (t,2H); 3.94 (t,2H); 5.74 (s,1H); 6.20 à 6.26 (m, 2H); 6.56 à 6.69 (m, 1H); 6.96 (s,1H); 7.26 (s,1H).

### EXEMPLE 2: (E, E, Z)-7-[3-(5-Hydroxy-pentyloxy)-5,5,8,8-tétraméthyl-5,6,7,8-tétrahydro-naphthalène-2-yl]-3-méthyl-octa-2,4,6-triènoate d'éthyle de formule

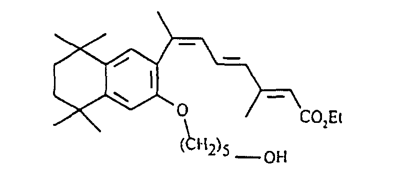

Un mélange de (E, E, Z)-7-{3-[5-(*tert*-butyl-diméthyl-silanyloxy)-pentyloxy]-5,5,8,8-tétraméthyl-5,6,7,8-tétrahydro-naphthalène-2-yl}-3-méthyl-octa-2,4,6-triènoate d'éthyle (250mg), d'une solution 1M dans le THF de fluorure de tétrabutylammonium (TBAF) (0,85ml) dans le THF (5ml) est agité à température ambiante pendant 1 h puis traité à l'acétate d'éthyle et à l'eau. Après décantation la phase organique est lavée deux fois à l'eau, séchée sur sulfate de magnésium anhydre et concentrée à l'évaporateur rotatif sous vide à 40°C.
Huile incolore, Rendement: 35%.
RMN d ppm: 1H/CDCl₃: 1.17 à 1.26 (m, 15H); 1.39 à 1.46 (m, 4H); 1.58 (s, 4H); 1.63 à 1.74 (m, 2H); 2.04 (s, 3H); 2.08 (s, 3H); 3.55 (t, 2H); 3.85 (t, 2H); 4.04 (q, 2H); 5.63(s,1H); 6.08 à 6.14 (m, 2H); 6.44 à 6.55 (m,1H); 6.69 (s, 1H); 6.87 (s, 1H).

## Revendications

1. Composés de formule générale (I) 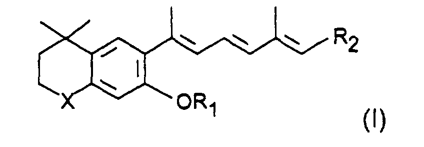 dans laquelle
X représente un radical divalent >C(CH₃)₂, un atome d'oxygène (-O-) ou un atome de soufre (-S-),
R₁ représente un radical de formule -(CH₂)ₙ-Z-(CH₂)ₒ-Y, Z, Y, n et o étant définis ci-dessous,
Y représente OH, un radical -thiol (-SH), un radical triméthylammonium (-N⁺(CH₃)₃), un radical cyano (-CN), un radical -COR₃, ou un radical -NH2, R₃ étant défini ci-dessous,
Z représente un radical méthylène (-CH₂-) ou un atome d'oxygène (-O-),
R₂ représente un radical -COR₃ ou -CH₂-OH,
R₃ représente un atome d'hydrogène, un radical hydroxyle, un radical alkoxyle ayant de 1 à 6 atomes de carbone ou cycloalkoxyle, ou encore un radical de formule -NR₄R₅,
R₄ et R₅, identiques ou différents, représentent indépendamment un atome d'hydrogène ou un radical alkyle ayant de 1 à 6 atomes de carbone, ou R₄ et R₅ forment ensembles un hétérocycle avec l'atome d'azote auquel ils sont rattachés,
n et o, identiques ou différents, représentent indépendamment un entier compris entre 1 et 5,
leurs racémiques ou leurs isomères optiques ou géométriques purs ou leurs mélanges en toutes proportions, et leurs sels pharmaceutiquement acceptables.

2. Composé selon la revendication 1, caractérisée en ce que Z représente un atome d'oxygène (-O-), Y représente un radical cyano (-CN) ou un radical -COR₃, R₃ étant défini dans la revendication 1, n représente 2, 3 ou 4 et o représente 1, 2 ou 3.

3. Composé selon la revendication 1, caractérisé en ce que Z représente un radical méthylène (-CH₂-) et Y représente un radical -thiol (-SH), triméthylammonium (-N⁺(CH₃)₃) ou cyano (-CN).

4. Médicament contentant les composés de formule générale (I) selon l'une des revendications 1 à 3.

5. Médicament selon la revendication 4, pour le traitement de désordres ou d'affections liés à une surrégulation des facteurs de transcription dénommés les récepteurs rétinoïdes qui comprennent les Récepteurs Rétinoïques X (RXRs), y compris les sous-types α, β, γ, ou les gènes comprenant les éléments de réponse RXRs.

6. Utilisation d'un composé selon l'une quelconque des revendications 1 à 3 pour la fabrication d'une composition pharmaceutique destinée au traitement des pathologies ou désordres suivants :
- l'acné; et/ou
- les affections dermatologiques liées à un trouble de la kératinisation avec une composante inflammatoire et/ou immunoallergique et notamment toutes les formes de psoriasis qu'il soit cutané, muqueux ou unguéal, et même le rhumatisme psoriatique ; et/ou
- certaines affections inflammatoires ne présentant pas de trouble de la kératinisation, telles que l'arthrite; et/ou
- le vieillissement de la peau, qu'il soit photo-induit ou chronologique ou les pigmentations et les kératoses actiniques, ou toutes pathologies associées au vieillissement chronologique ou actinique ; et/ou
- les troubles de la fonction sébacée tels que l'hyperséborrhée de l'acné ou la séborrhée simple; et/ou
- les états cancéreux ou précancéreux; et/ou
- les proliférations dermiques ou épidermiques malignes; et/ou
- les affections d'origine virale au niveau cutané ou général (virus d'immunodéficience humain : HIV-1 ou virus de l'hépatite B) ; et/ou
- l'alopécie; et/ou
- les affections du système cardio-vasculaire.

7. Composition cosmétique ou pharmaceutique, caractérisée en ce qu'elle comprend au moins un composé de formule générale (I) selon l'une des revendications 1 à 3 et au moins un véhicule cosmétiquement ou pharmaceutiquement acceptable.

8. Composition selon la revendication 7, pour l'administration des composés de formule générale (I) selon l'une des revendications 1 à 3 par voie entérale, parentérale, topique ou oculaire.

9. Composition selon l'une des revendications 7 ou 8, à usage topique ou oculaire.

10. Composition selon la revendication 9, caractérisée en ce qu'elle contient au moins un composé de formule (I) tel que défini dans les revendications 1 à 3, à une concentration comprise entre 0,001% et 5% en poids par rapport au poids total de la composition.

## Patentansprüche

1. Verbindungen der allgemeinen Formel (I) 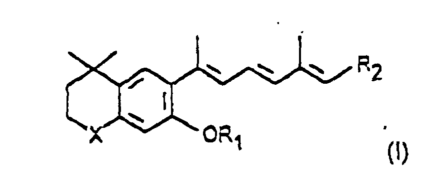 worin bedeuten:
- X eine zweiwertige Gruppe >C(CH₃)₂, ein Sauerstoffatom (-O-) oder ein Schwefelatom (-S-),
- R₁ eine Gruppe der Formel -(CH₂)ₙ-Z-(CH₂)ₒ-Y, wobei Z, Y, n und o nachfolgend definiert werden,
- Y OH, eine Thiogruppe (-SH), eine Trimethylammoniumgruppe (-N⁺(CH₃)₃), eine Cyanogruppe (-CN), eine Gruppe -COR₃ oder eine Gruppe -NH₂, wobei R₃ nachfolgend definiert wird,
- Z eine Methylengruppe (-CH₂-) oder ein Sauerstoffatom (-O-),
- R₂-COR₃ oder -CH₂-OH,
- R₃ ein Wasserstoffatom, eine Hydroxygruppe, eine Alkoxygruppe mit 1 bis 6 Kohlenstoffatomen, eine Cycloalkoxygruppe oder eine Gruppe der Formel -NR₄R₅,
- R₄ und R₅, die identisch oder voneinander verschieden sind, unabhängig voneinander Wasserstoff oder eine Alkylgruppe mit 1 bis 6 Kohlenstoffatomen oder die Gruppen R₄ und R₅ bilden gemeinsam mit dem Stickstoffatom, an das sie gebunden sind, einen Heterocyclus,
- n und o, die identisch oder voneinander verschieden sind, unabhängig voneinander ganze Zahlen im Bereich von 1 bis 5, ihre Racemate oder ihre reinen optischen oder geometrischen Isomere oder deren Gemische in beliebigen Mengenanteilen und ihre pharmazeutisch akzeptablen Salze.

2. Verbindungen nach Anspruch 1, dadurch gekennzeichnet, daß Z ein Sauerstoffatom (-O-), Y eine Cyanogruppe (-CN) oder eine Gruppe -COR₃, wobei R₃ die in Anspruch 1 angegebenen Bedeutungen aufweist, n 2, 3 oder 4 und o 1, 2 oder 3 bedeuten.

3. Verbindungen nach Anspruch 1, dadurch gekennzeichnet, daß Z eine Methylengruppe (-CH₂-) ist und Y eine Thiogruppe (-SH), eine Trimethylammoniumgruppe (-N⁺(CH₃)₃) oder eine Cyanogruppe (-CN) bedeutet.

4. Arzneimittel, das die Verbindungen der Formel (I) nach einem der Ansprüche 1 bis 3 enthält.

5. Arzneimittel nach Anspruch 4 zur Behandlung von Störungen oder Erkrankungen, die mit einer Überregulierung von als Retinoidrezeptoren bezeichneten Transkriptionsfaktoren, die die Retinoidrezeptoren X (RXRs) einschließlich der Untertypen α, β, y umfassen, oder von Genen, die RXR-Responsivelemente enthalten, zusammenhängen.

6. Verwendung einer Verbindung nach einem der Ansprüche 1 bis 3 zur Herstellung einer pharmazeutischen Zusammensetzung, die zur Behandlung der folgenden Erkrankungen oder Störungen vorgesehen ist:
- Akne; und/oder
- dermatologische Erkrankungen, die mit einer Keratinisierungsstörung mit einer entzündlichen und/oder immunoallergischen Komponente zusammenhängen, insbesondere beliebige Formen der Psoriasis der Haut, der Schleimhäute oder der Nägel, oder auch arthropathische Psoriasis; und/oder
- verschiedene entzündliche Erkrankungen ohne Keratinisierungsstörung, beispielsweise Arthrtitis; und/oder
- Hautalterung, die lichtinduziert oder altersbedingt sein kann, aktinische Pigmentierungen und Keratosen oder beliebige Erkrankungen, die mit einer altersbedingten oder aktinischen Hautalterung verbunden sind; und/oder
- Funktionsstörungen der Talgdrüsen, wie Hyperseborrhoe bei Acne oder Seborrhoe simplex; und/oder
- canceröse oder präcanceröse Zustände; und/oder
- maligne Proliferationen der Dermis oder Epidermis; und/oder
- Hauterkrankungen viralen Ursprungs oder allgemeine Erkrankungen viralen Ursprungs (menschliches Immunschwächevirus HIV-1 oder Hepatitis B Virus); und/oder
- Alopezie; und/oder
- Erkrankungen des cardiovaskulären Systems.

7. Kosmetische oder pharmazeutische Zusammensetzung, dadurch gekennzeichnet, daß sie mindestens eine Verbindung der allgemeinen Formel (I) nach einem der Ansprüche 1 bis 3 und mindestens einen kosmetisch oder pharmazeutisch akzeptablen Träger enthält.

8. Zusammensetzung nach Anspruch 7 zur enteralen, parenteralen, topischen oder okularen Verabreichung von Verbindungen der allgemeinen Formel (I) nach einem der Ansprüche 1 bis 3.

9. Zusammensetzung nach einem der Ansprüche 7 oder 8 zur topischen oder okularen Anwendung.

10. Zusammensetzung nach Anspruch 9, dadurch gekennzeichnet, daß sie mindestens eine Verbindung der Formel (I) nach einem der Ansprüche 1 bis 3 in einer Konzentration von 0,001 bis 5 Gew.-%, bezogen auf das Gesamtgewicht der Zusammensetzung, enthält.

## Claims

1. Compounds of general formula (I) 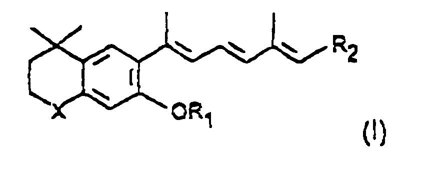 in which
X represents a bivalent radical >C(CH₃)₂, an oxygen atom (-O-) or a sulphur atom (-S-),
R₁ represents a radical of formula -(CH₂)ₙ-Z-(CH₂)₀-Y, Z, Y, n and o being defined below,
Y represents OH, a thiol radical (-SH), a trimethylammonium radical (-N⁺(CH₃)₃), a cyano radical (-CN), a radical -COR₃, or an -NH₂ radical, R₃ being defined below,
Z represents a methylene radical (-CH₂-) or an oxygen atom (-O-),
R₂ represents a radical -COR₃ or -CH₂-OH,
R₃ represents a hydrogen atom, a hydroxyl radical, an alkoxy radical having from 1 to 6 carbon atoms or a cycloalkoxy radical, or alternatively a radical of formula -NR₄R₅,
R₄ and R₅, which may be identical or different, independently represent a hydrogen atom or an alkyl radical having from 1 to 6 carbon atoms, or R₄ and R₅ together form a heterocycle with the nitrogen atom to which they are attached,
n and o, which may be identical or different, independently represent an integer between 1 and 5, their racemates or their pure optical or geometric isomers or mixtures thereof in all proportions, and their pharmaceutically acceptable salts.

2. Compound according to Claim 1, characterized in that Z represents an oxygen atom (-O-) and Y represents a cyano radical (-CN) or a radical -COR₃, R₃ being defined in Claim 1, n represents 2, 3 or 4 and o represents 1, 2 or 3.

3. Compound according to Claim 1, characterized in that Z represents a methylene radical (-CH₂-) and Y represents a thiol (-SH), trimethylammonium (-N⁺(CH₃)₃) or cyano (-CN) radical.

4. Medicament containing the compounds of general formula (I) according to one of Claims 1 to 3.

5. Medicament according to Claim 4, for the treament of disorders or complaints linked to an overregulation of the transcription factors designated retinoid recaptors which comprise the retinoic X receptors (RXRs), including the subtypes α, β and γ, or the genes comprising the RXR response elements,

6. Use of a compound according to any one of Claims 1 to 3, for the manufacture of a pharmaceutical composition for treating the following pathologies or disorders:
- acne; and/or
- dermatological complaints linked to a keratinization disorder with an inflammatory and/or immunoallergic component, and in particular all forms of psoriasis, whether cutaneous, mucosal or ungual, and even arthropathia psotiatica; and/or
- some inflammatory complaints not entailing a keratinization disorder, such as arthritis; and/or
- skin aging, whether light-induced or natural, or actinic keratosís and pigmentations, or all pathologies associated with natural or actinic aging; and/or
- disorders of sebaceous function, such as hyperseborrhoea associated with acne or simple seborrhoea; and/or
- cancerous or precancerous conditions; and/or
- malignant dermal or epidermal proliferations; and/or
- cutaneous or systemic complaints of viral origin (human immunodeficiency virus: HIV-1, or hepatitis B virus); and/or
- alopecia; and/or
- complaints of the cardiovascular system.

7. Cosmetic or pharmaceutical composition, characterized in that it comprises at least one compound of general formula (I) according to one of Claims 1 to 3 and at least one cosmetically or pharmaceutically acceptable vehicle.

8. Composition according to Claim 7, for administration of the compounds of general formula (I) according to one of Claims 1 to 3 via the enteral, parenteral, topical or ocular route.

9. Composition according to either of Claims 7 and 8, for topical or ocular application.

10. Composition according to Claim 9, characterized in that it contains at least one compound of formula (I) as defined in Claims 1 to 3, at a concentration of between 0.001% and 5% by weight relative to the total weight of the composition.
